# Europäisches Patentamt
# European Patent Office
# Office européen des brevets

(19)

(11) Veröffentlichungsnummer: **0 126 351**
**B1**

(12) **EUROPÄISCHE PATENTSCHRIFT**

(45) Veröffentlichungstag der Patentschrift:
01.04.87

(51) Int. Cl.⁴: **C 07 D 499/18**, C 07 D 307/94, C 07 C 69/675

(21) Anmeldenummer: **84105016.4**

(22) Anmeldetag: **04.05.84**

(54) Verfahren zur Isolierung und Reinigung von Antibiotika.

(30) Priorität: **19.05.83 DE 3318194**

(43) Veröffentlichungstag der Anmeldung:
**28.11.84 Patentblatt 84/48**

(45) Bekanntmachung des Hinweises auf die Patenterteilung:
**01.04.87 Patentblatt 87/14**

(84) Benannte Vertragsstaaten:
**AT BE CH FR GB IT LI NL SE**

(56) Entgegenhaltungen:
**DE - A - 3 218 597**
**FR - A - 1 439 587**
**FR - A - 1 462 217**
**GB - A - 1 341 921**
**US - A - 4 089 891**
**US - A - 4 354 971**

(73) Patentinhaber: **Fried. Krupp Gesellschaft mit beschränkter Haftung, Altendorfer Strasse 103, D-4300 Essen 1 (DE)**

(72) Erfinder: **Coenen, Hubert, Dr., Wortbergrode 13, D-4300 Essen 1 (DE)**
Erfinder: **Hagen, Rainer, Schäferstrasse 24, D-4300 Essen (DE)**

## Beschreibung

Die Erfindung bezieht sich auf ein Verfahren zur Isolierung und Reinigung von Antibiotika. Die Antibiotika bilden eine Gruppe von Arzneimitteln, die sich zur Bekämpfung von Infektionskrankheiten eignen. Die Antibiotika werden meistens durch einen Mikrogrganismus produziert und sind in der Lage, andere Mikroorganismen in ihrem Wachstum zu hemmen oder abzutöten. Zur Gruppe der Antibiotika gehören die Penicilline, Cephalosporine, Tetracycline, Aminoglykosid-Antibiotika, Nukleosid-Antibiotika, Makrolid-Antibiotika, Ansamycine, Peptid-Antibiotika und Antibiotika mit individueller Struktur. Die weitaus meisten Antibiotika werden in einem Fermentationsprozess unter sterilen Bedingungen von einem Mikroorganismus gebildet, der in einer Nährlösung unter Zufuhr von Luft kultiviert wird. Wenn die Kultur einen ausreichend hohen Gehalt des vom Mikroorganismus gebildeten Antibiotikums hat, wird sie abgeerntet, was entweder durch Trocknung, insbesondere Sprühtrocknung, der gesamten Kultur oder durch Filtration und anschliessende Aufarbeitung der antibiotikahaltigen Kulturlösung bzw. des antibiotikahaltigen Mycels erfolgt. Die Aufarbeitung der antibiotikahaltigen Kulturlösung bzw. des antibiotikahaltigen Mycels erfolgt durch vielstufige Isolierungs- und Reinigungsverfahren, wobei in einer ersten Stufe eine Extraktion und in einer zweiten Stufe eine Fällung der extrahierten Antibiotika durchgeführt wird. Es ist auch möglich, die Antibiotika in der ersten Verfahrensstufe direkt aus der abfiltrierten antibiotikahaltigen Kulturlösung auszufällen. Die durch Fällung erhaltenen Zwischenprodukte müssen gereinigt werden, was insbesondere durch mehrmaliges Umkristallisieren und Umfällen geschieht. Da sich viele Antibiotika aus unterschiedlichen Gründen im gelösten Zustand schnell zersetzen, muss ihre Isolierung und Reinigung möglichst schnell erfolgen. Ausserdem ist bei der Isolierung und Reinigung von Antibiotika darauf zu achten, dass sie nicht zu stark erwärmt werden, da sich die meisten dieser Verbindungen bei höheren Tamperaturen zersetzen.

Bei den bekannten Verfahren, die zur Isolierung und Reinigung der Antibiotika angewendet werden, fallen in den einzelnen Verfahrensstufen antibiotikahaltige Lösungen an, die neben Wasser auch organische Lösungsmittel enthalten. Aus diesen Lösungen müssen nicht nur die Antibiotika abgetrennt, sondern auch die organischen Lösungsmittel entfernt werden, bevor sie in das Abwasser eingeleitet werden können. Ausserdem verursacht die Vielstufigkeit der Isolierungs- und Reinigungsverfahren Ausbeuteverluste sowie Betriebsstörungen.

Der Erfindung liegt daher die Aufgabe zugrunde, ein Verfahren zur Isolierung und Reinigung von Antibiotika zu schaffen, das die Zahl der erforderlichen Verfahrensschritte signifikant verringert und die Antibiotika-Ausbeute erhöht.

Die der Erfindung zugrundeliegende Aufgabe wird dadurch gelöst, dass eine antibiotikahaltige Lösung mit einem Extraktionsmittel, dessen kritische Temperatur im Bereich von 0 bis 160°C liegt, bei einer Temperatur von 0 bis 40°C sowie einem Druck, der zwischen dem kritischen Druck und dem zahnfachen Wert des kritsichen Drucks des Extraktionsmittels liegt, extrahiert wird, wobei das Gewichtsverhältnis von antibiotikahaltiger Lösung zum Extraktionsmittel 1:5 bis 1:20 beträgt, dass das antibiotikahaltige Extraktionsmittel anschliessend von der extrahierten Lösung abgetrennt wird, dass die Antibiotika danach aus dem antibiotikahaltigen Extraktionsmittel durch Erniedrigung seiner Dichte ausgefällt werden und dass das Extraktionsmittel dann in die Extraktionsstufe zurückgeführt wird. Dieses Verfahren arbeitet in vorteilhafter Weise bei Temperaturen, die keine Zersetzung der Antibiotika bewirken. Ferner liefert das Verfahren sehr reine Antibiotika, wodurch ein mehrmaliges Umkristallisieren bzw. Umfällen entfallen kann. Schliesslich erfolgt die Ausfällung der Antibiotika aus dem antibiotikahaltigen Extraktionsmittel durch Erniedrigung seiner Dichte, was den Vorteil hat, dass zusätzliche Fällungsmittel nicht benötigt werden.

In weiterer Ausgestaltung der Erfindung ist vorgesehen, dass als Extraktionsmittel $CO_2$, $C_2H_4$, $C_2H_6$, $C_3H_6$, $C_3H_8$, $C_4H_{10}$, $SF_6$, $CHClF_2$, $CF_3Cl$, $CF_3Br$, $C_2F_2Cl_2H_2$, $N_2O$ oder ein Gemisch aus mindestens zwei dieser Stoffe verwendet wird. Diese Extraktionsmittel haben den Vorteil, dass die rückstandsfrei aus der zu extrahierenden Lösung entfernt werden können oder doch im extrahierten Lösungsmittel verbleiben dürfen (z.B. Kohlendioxid). Ferner ist nach der Erfindung vorgesehen, dass dem Extraktionsmittel 1 bis 50 Gew.-% Äthanol, Methylacetat, Äthylacetat, Propylacetat, Butylacetat, Diäthyläther, Aceton, Methylenchlorid und/oder Wasser als Schleppmittel zugesetzt wird. Durch die Verwendung eines Schleppmittels kann die Löslichkeit des Extraktionsmittels für die zu extrahierenden Antibiotika so erhöht werden, dass eine wesentliche Verkürzung der Extraktionszeit erreicht wird. Die Schleppmittel müssen sowohl aus der extrahierten Lösung als auch aus dem Extrakt zurückgewonnen werden.

Nach der Erfindung ist es besonders vorteilhaft, wenn die Erniedrigung der Dichte des antibiotikahaltigen Extraktionsmittels durch Entspannung auf einen Druck von 30 bis 70 bar bei einer Temperatur von 0 bis 40°C erfolgt, denn bei dieser Temperatur tritt eine Zersetzung der Antibiotika nicht ein. In manchen Fällen ist es besonders vorteilhaft, wenn die Entspannung des antibiotikahaltigen Extraktionsmittels in mehreren Stufen erfolgt, da mit dieser Verfahrensführung mehrere Antibiotika-Fraktionen unterschiedlicher Reinheit gewonnen werden können, von denen die mit geringerem Reinheitsgrad im Kreislauf zu führen sind.

Obwohl aus der DE-AS 1 493 190 ein Verfahren zur Auftrennung von flüssigen und/oder festen Stoffgemischen bekannt ist, bei dem das Stoffgemisch mit einem unter überkritischen Bedingungen der Temperatur des Drucks stehenden Gas im Temperaturbereich bis über 100°C über seiner kritischen Temperatur behandelt wird und nach Abtrennung der beladenen überkritischen Gasphase die in ihr enthaltenen Verbindungen durch Entspannung und/oder Temperaturerhöhung zurückgewonnen werden, kann diese Druckschrift den Fachmann nicht zum erfindungsgemässen Verfahren hinführen, denn sie enthält keinen Hinweis darauf, dass die Antibiotika mit einem Extraktionsmittel, dessen kritische Temperatur im Bereich von 0 bis 160°C liegt, bei einer Temperatur von 0 bis 40°C sowie einem Druck, der zwischen dem kritischen Druck und dem zehnfachen Wert des kritischen Drucks des Extraktionsmittels liegt, extrahiert werden können und dass die Ausfällung der Antibiotika bei einer Temperatur von 0 bis 40°C erfolgen kann.

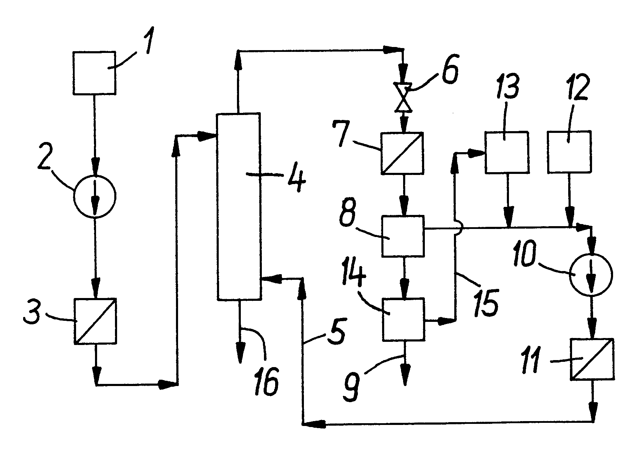

Der Gegenstand der Erfindung wird nachfolgend anhand der Zeichnung und von mehreren Ausführungsbeispielen näher erläutert.

Die Zeichnung zeigt den prinzipiellen Ablauf des erfindungsgemässen Verfahrens, der jeweils den speziellen Erfordernissen des Einzelfalles anzupassen ist. Die ungereinigte antibiotikahaltige Lösung gelangt aus dem Vorratstank 1 in eine Pumpe 2, von der sie auf den Extraktionsdruck komprimiert und in den Wärmeaustauscher 3 gefördert wird, wo die Einstellung der Extraktionstemperatur erfolgt. Die Lösung wird danach in den Kopfteil einer Hochdruckkolonne 4 gefördert, der am Fuss über die Leistung 5 das Extraktionsmittel zugeführt wird. In der Hochdruckkolonne 4, die den Stoffaustausch begünstigende Einbauten enthält, wird die antibiotikahaltige Lösung vom Extraktionsmittel im Gegenstrom kontinuierlich extrahiert, wobei die Verunreinigungen in der Lösung verbleiben. Das antibiotikahaltige Extraktionsmittel wird der Hochdruckkolonne 4 kopfseitig entnommen und über das Entspannungsventil 6 sowie den Wärmeaustauscher 7 in den Abscheider 8 gefördert, wo die Antibiotika ausfallen. Wenn das Extraktionsmittel ein Schleppmittel enthält, fällt ein Teil des Schleppmittels zusammen mit den Antibiotika aus. In der nachgeschalteten Separierstufe 14 werden die Antibiotika vom Schleppmittel getrennt. Die Antibiotika werden über die Leitung 9 entnommen, während das Schleppmittel über die Leitung 15 in den Vorratstank 13 gelangt. Die Antibiotika haben in den meisten Fällen eine aureichende Reinheit und werden lediglich nachgetrocknet sowie sterilisiert. Aus dem Abscheider 8 wird das antibiotikafreie Extraktionsmittel in die Pumpe 10 geführt, die es auf den Extraktionsdruck komprimiert und in den Wärmeaustauscher 11 fördert, wo die Einstellung der Extraktionstemperatur erfolgt. Danach galangt das Extraktionsmittel in die Hochdruckkolonne 4. Extraktionsmittelverluste werden aus dem Vorratsschrank 12 ergänzt. Die dem Extraktionsmittel zuzusetzenden Schleppmittel werden dem Vorratstank 13 entnommen. Der Extraktionsrückstand wird der Hochdruckkolonne 4 über die Leitung 6 entnommen. Es handelt sich dabei um die antibiotikafreie bzw. antibiotikaarme extrahierte Lösung, die noch Schleppmittelreste enthalten kann.

Nach dem erfindungsgemässen Verfahren wurden mehrere Antibiotika aus unterschiedlich zusammengesetzten antibiotikahaltigen Lösungen in reiner Form isoliert.

Beispiel 1

Eine ungereinigte Lösung, die aus 72,2% Äthanol, 23,8% Wasser und 4% Penicillin-V-Säure (= α-Phenoxyäthyl-Penicillinsäure) besteht, wird kontinuierlich mit Kohlendioxid bei 200 bar und 37°C extrahiert. Dabei nimmt 1l Gasphase 6 g Penicillin-V-Säure auf und löst ausserdem Äthanol und Wasser entsprechend den thermodynamischen Gleichgewichtsbedingungen. Die Penicillin-V-Säure wird aus dem Extraktionsmittel durch Entspannung auf 60 bar bei 28°C gewonnen. Eine HPLC-Analyse zeigt, dass die isolierte Penicillin-V-Säure eine ausserordentlich hohe Reinheit hat und dass die in der antibiotikahaltigen Lösung vorhandenen Begleitstoffe bei der Extraktion nahezu quantitativ im Extraktionsrückstand verbleiben.

Beispiel 2

Eine ungereinigte Lösung, die aus 42,5% Äthanol, 40,5% Wasser und 17% Penicillin-V-Säure besteht, wird kontinuierlich mit Kohlendioxid bei 200 bar und 37°C extrahiert. Bei der Extraktion nimmt 1 l Gasphase 3,5 g Penicillin-V-Säure auf und löst daneben Äthanol sowie Wasser entsprechend den thermodynamischen Gleichgewichtsbedingungen. Aus dem Extraktionsmittel wird die Penicillin-V-Säure durch Entspannung auf 60 bar bei 28°C abgeschieden. Das so gewonnene Produkt hat eine sehr hohe Reinheit und ist frei von den in der Ausgangslösung vorhandenen Begleitstoffen, was mit einer HPLC-Analyse nachgewiesen wurde.

Beispiel 3

Eine ungereinigte Lösung, die aus 65% Äthanol, 33,5% Wasser und 1,5% Penicillin-V-Säure besteht, wird kontinuierlich mit Äthan bei 200 bar und 37°C extrahiert. Bei der Extraktion wird von 1l Gasphase 0,9 g Penicillin-V-Säure ausgetragen, und ausserdem nimmt das Extraktionsmittel Äthanol sowie Wasser entsprechend den thermodynamischen Gleichgewichtsbedingungen auf. Die Penicillin-V-Säure wird durch Entspannung auf 40 bar bei 28°C aus dem Extraktionsmittel abgeschieden. Das Produkt hat eine sehr hohe Reinheit und ist weitgehend frei von den in der Ausgangslösung vorhandenen Begleitstoffen, was durch eine HPLC-Analyse nachgewiesen werden konnte.

Beispiel 4

Eine ungereinigte Lösung, die 99% Wasser, 0,6% Äthylacetat und 0,4% Penicillin-V-Säure enthält, wird kontinuierlich mit Kohlendioxid bei 300 bar und 37°C extrahiert. Bei der Extraktion wird von 1l Gasphase 1,2 g Penicillin-V-Säure aufgenommen und ausserdem Wasser sowie Äthylacetat entsprechend den thermodynamischen Gleichgewichtsbedingungen gelöst. Die Penicillin-V-Säure wird durch Entspannung auf 60 bar bei 28°C aus dem Extraktionsmittel abgeschieden. Das so gewonnene Produkt hat eine sehr hohe Reinheit, und die in der Ausgangslösung vorhandenen Begleitstoffe verbleiben nahezu quantitativ im Extraktionsrückstand, was durch eine HPLC-Analyse nachgewiesen wurde.

### Beispiel 5

Eine ungereinigte Lösung, die aus 93,2% Äthanol, 4,0% Wasser und 2,8% Pleuromulin besteht, wird kontinuierlich mit Äthan bei 200 bar und 37°C extrahiert. 1l Gasphase nimmt während der Extraktion 8 g Pleuromulin sowie Wasser und Äthanol entsprechend den thermodynamischen Gleichgewichten auf. Das Pleuromulin wird durch Entspannung auf 40 bar bei 28°C aus dem Extraktionsmittel abgeschieden. Das so gewonnene Endprodukt hat eine sehr hohe Reinheit.

### Beispiel 6

Eine ungereinigte Lösung, die aus 75,4% Äthanol, 23,4% Wasser und 1,2% Griseofulvin besteht, wird kontinuierlich mit Äthan bei 200 bar und 37°C extrahiert. 1l Gasphase nimmt während der Extraktion 0,3 g Griseofulvin und daneben entprechend den thermodynamischen Gleichgewichten Wasser sowie Äthanol auf. Das Griseofulvin wird durch Entspannung auf 40 bar bei 28°C aus dem Extraktionsmittel abgetrennt. Das Endprodukt hat eine hohe Reinheit, was mit einer HPLC-Analyse nachgewiesen wurde.

Bei den in den Ausführungsbeispielen 1 bis 6 genannten Prozentzahlen handelt es sich um Gew.-%.

### Patentansprüche

1. Verfahren zur Isolierung und Reinigung von Antibiotika, dadurch gekennzeichnet, dass eine antibiotikahaltige Lösung mit einem Extraktionsmittel, dessen kritische Temperatur im Bereich von 0 bis 160°C liegt, bei einer Temperatur von 0 bis 40°C sowie einem Druck, der zwischen dem kritischen Druck und dem zehnfachen Wert des kritischen Drucks des Extraktionsmittels liegt, extrahiert wird, wobei das Gewichtsverhältnis von antibiotikahaltiger Lösung zum Extraktionsmittel 1:5 bis 1:20 beträgt, dass das antibiotikahaltige Extraktionsmittel anschliessend von der extrahierten Lösung abgetrennt wird, dass die Antibiotika danach aus dem antibiotikahaltigen Extraktionsmittel durch Erniedrigung seiner Dichte ausgefällt werden und dass das Extraktionsmittel dann in die Extraktionsstufe zurückgeführt wird.

2. Verfahren nach Anspruch 1, dadurch gekennzechnet, dass als Extraktionsmittel $CO_2$, $C_2H_4$, $C_2H_6$, $C_3H_6$, $C_3H_8$, $C_4H_{10}$, $SF_6$, $CHClF_2$, $CF_3Cl$, $CF_3Br$, $C_2F_2Cl_2H_2$, $N_2O$ oder ein Gemisch aus mindestens zwei dieser Stoffe verwendet wird.

3. Verfahren nach den Ansprüchen 1 und 2, dadurch gekennzeichnet, dass dem Extraktionsmittel 1 bis 50 Gew.-% Äthanol, Methylacetat, Äthylacetat, Propylacetat, Butylacetat, Diäthyläther, Aceton, Methylenchlorid und/oder Wasser als Schleppmittel zugesetzt wird.

4. Verfahren nach den Ansprüchen 1 bis 3, dadurch gekennzeichnet, dass die Erniedrigung der Dichte des antibiotikahaltigen Extraktionsmittels durch Entspannung auf einen Druck von 30 bis 70 bar bei einer Temperatur von 0 bis 40°C erfolgt.

5. Verfahren nach den Ansprüchen 1 bis 4, dadurch gekennzeichnet, dass die Entspannung des antibiotikahaltigen Extraktionsmittels in mehreren Stufen erfolgt.

### Claims

1. Process for the isolation and purification of antibiotics, characterised in that an antibiotic-containing solution is extracted with an extraction agent the critical temperature of which lies in the range of 0 to 160°C, at a temperature of 0 to 40°C and a pressure lying between the critical pressure and ten times the value of the critical pressure of the extraktion agent, the weight ratio of antibiotic-containing solution to extraktion agent amounting to 1:5 to 1:20, in that the antibiotic-containing extraction agent is then separated from the extracted solution, in that the antibiotics are then precipitated out of the antibiotic-containing extraxtion agent by reduction of its density and in that the extraction agent is then returned to the extraction stage.

2. Process according to Claim 1, characterised in that as extraction agent there is used $CO_2$, $C_2H_4$, $C_2H_6$, $C_3H_6$, $C_3H_8$, $C_4H_{10}$, $SF_6$, $CHClF_2$, $CF_3Cl$, $CF_3Br$, $C_2F_2Cl_2H_2$, $N_2O$ or a mixture of at least two of these substances.

3. Process according to Claims 1 and 2, characterised in that 1 to 50% wt. of ethanol, methyl acetate, ethyl acetate, propyl acetate, butyl acetate, diethyl ether, acetone, methylene chloride and/or water is added as entrainer to the extraction agent.

4. Process according to Claims 1 to 3, characterised in that the reduction of the density of the antibiotic-containing extraction agent is effected by expansion to a pressure of 30 to 70 bars at a temperature of 0 to 40°C.

5. Process according to Claims 1 to 4, characterised in that the expansion of the antibiotic-containing extraction agent takes place in several stages.

### Revendications

1. Procédé pour isoler et purifier des antibiotiques, caractérisé en ce qu'une solution contenant des antibiotiques, est soumise à une extraction, au moyen d'un produit d'extraction dont la température critique est de l'ordre de 0 à 160°C, à une température de 0 à 40°C et sous une pression comprise entre la pression critique du produit d'extraction et une pression dix fois supérieure à cette pression critique, le rapport des poids entre la solution contenant les antibiotiques et le produit d'extraction étant de 1:5 à 1:20, en ce que le produit d'extraction chargé d'antibiotiques est ensuite séparé de la solution qui a été soumise à l'extraction, en ce qu'ensuite les antibiotiques sont précipités à partir du produit d'extraction du fait que sa densité est diminuée et en ce que le produit d'extraction est ensuite ramené à l'étage d'extraction.

2. Procédé selon la revendication 1, caractérisé en ce que, comme produits d'extraction, on utilise $CO_2$, $C_2H_4$, $C_2H_6$, $C_3H_6$, $C_3H_8$, $C_4H_{10}$, $SF_6$, $CHClF_2$, $CF_3Cl$, $CF_3Br$, $C_2F_2Cl_2H_2$, $N_2O$ ou un mélange de deux au moins de ces substances.

3. Procédé selon les revendications 1 et 2, caractérisé en ce qu'on ajoute au praduit d'extraction, comme produit d'entraînement, 1 à 50% en poids d'éthanol, d'acétate de méthyle, d'acétate d'éthyle, d'acétate de propyle, d'acétate de butyle, de diéthyl-éther, d'acétone, de chlorure de méthylène et/ou d'eau.

4. Procédé selon les revendications 1 à 3, caractérisé en ce que la diminution de la densité du produit d'extraction chargé d'antibiotiques s'obtient par détente à une pression de 30 à 70 bars à une température de 0 à 40°C.

5. Procédé selon les revendications 1 à 4, caractérisé en ce que la détente du produit d'extraction chargé d'antibiotiques s'effectue en plusieurs stades.